**Europäisches Patentamt**

**European Patent Office**

**Office européen des brevets**

(19)

(11) Veröffentlichungsnummer : **0 059 414**
**B1**

(12) **EUROPÄISCHE PATENTSCHRIFT**

(45) Veröffentlichungstag der Patentschrift :
**30.10.85**

(21) Anmeldenummer : **82101359.6**

(22) Anmeldetag : **23.02.82**

(51) Int. Cl.⁴ : **B 01 J 23/22, C 07 D213/85**

(54) **Katalysatoren für die Herstellung von 3-Cyanpyridin.**

(30) Priorität : **28.02.81 DE 3107755**

(43) Veröffentlichungstag der Anmeldung :
**08.09.82 Patentblatt 82/36**

(45) Bekanntmachung des Hinweises auf die Patenterteilung : **30.10.85 Patentblatt 85/44**

(84) Benannte Vertragsstaaten :
**AT BE CH DE FR GB IT LI NL SE**

(56) Entgegenhaltungen :
**DE-A- 1 518 697**
**DE-A- 2 039 497**
**DE-A- 2 435 344**
**US-A- 4 051 140**

(73) Patentinhaber : **Degussa Aktiengesellschaft**
**Weissfrauenstrasse 9**
**D-6000 Frankfurt am Main 1 (DE)**

(72) Erfinder : **Beschke, Helmut**
**Greifenhagenstrasse 23**
**D-6450 Hanau 9 (DE)**
Erfinder : **Friedrich, Heinz, Dr.Dipl.-Chem.**
**Grünaustrasse 4**
**D-6450 Hanau 9 (DE)**
Erfinder : **Heilos, Johannes**
**Westring 16**
**D-6453 Seligenstadt (DE)**

## Beschreibung

Die Erfindung betrifft ein Verfahren zur Herstellung von 3-Cyanpyridin durch katalytische Umsetzung von 3-Methylpyridin mit Ammoniak und Sauerstoff bei erhöhter Temperatur. Sie betrifft insbesondere die für diesen Zweck geeigneten Katalysatoren aus Verbindungen der Elemente Antimon, Vanadin und Sauerstoff und wenigstens einem der Elemente Eisen, Kupfer, Titan, Kobalt, Mangan und Nickel, sowie das Verfahren zur Herstellung der Katalysatoren.

Es sind mehrere Verfahren zur Herstellung von 3-Cyanpyridin aus 3-Methylpyridin durch dessen Umsetzung mit Ammoniak und Sauerstoff bei erhöhter Temperatur in der Gasphase bekannt. Sie unterscheiden sich durch die Umsetzungsbedingungen und insbesondere durch die Katalysatoren. Unter den Verfahren und Katalysatoren sind nur diejenigen für eine Anwendung in technischem Maßstab von Bedeutung, die eine gute Selektivität aufweisen und gleichzeitig hohe Raum-Zeit-Ausbeuten ergeben.

Es ist bekannt, als Katalysator für die Umsetzung von Alkylpyridinen zu Cyanpyridinen Zinnphosphat mit Zusätzen von Verbindungen der Elemente Molybdän, Wismut, Vanadin, Eisen oder Kobalt zu verwenden (DE-B-1 770 841). Dieses Verfahren ergibt im Falle der Umsetzung von 3-Methylpyridin zu 3-Cyanpyridin nur mäßige Ausbeuten bei zudem geringer Selektivität.

Es ist außerdem bekannt, als Katalysatoren Zinnvanadat im Gemisch mit Diphosphorpentoxid auf Aluminiumoxid, Silikagel oder deren Gemischen (JP-A-426 066) oder Molybdänoxid im Gemisch mit Oxiden des Vanadins, Chroms, Mangans oder Kobalts auf Aluminiumoxid, Magnesiumoxid, Siliciumoxid oder Titanoxid (JP-A-4 513 572) oder reines Divanadinpentoxid bestimmter Oberfläche und Korngröße (DE-A-2 435 344) einzusetzen. Diese Verfahren ergeben zwar verhältnismäßig günstige Ausbeuten bei guter Selektivität, erfordern jedoch in starkem Maße durch Luft verdünnte Reaktionsgase. Es werden nur kleine Raum-Zeit-Ausbeuten erzielt.

Es ist weiter bekannt, Katalysatoren zu verwenden, die dadurch hergestellt werden, daß Mischungen, die Antimon und Vanadin im atomaren Verhältnis von 1,1 zu 1 bis 50 zu 1 und wenigstens eines der Elemente Eisen, Kupfer, Titan, Kobalt, Mangan und Nickel und gegebenenfalls eine Trägersubstanz enthalten, durch Erhitzen auf Temperaturen von 600 bis 1 100 °C in Gegenwart von Sauerstoff vorbehandelt werden (DE-C-2 039 497). Bei diesem Verfahren werden zwar hohe Raum-Zeit-Ausbeuten erzielt, die Selektivität der Katalysatoren ist jedoch unbefriedigend.

Es sind nun Katalysatoren für die Umsetzung von 3-Methylpyridin mit Ammoniak und Sauerstoff zu 3-Cyanpyridin, bestehend aus Verbindungen der Elemente Antimon, Vanadin und Sauerstoff und mindestens einem der Elemente Eisen, Kupfer, Titan, Kobalt, Mangan und Nickel, wobei das atomare Verhältnis Antimon zu Vanadin größer als 1 ist, gefunden worden, die dadurch gekennzeichnet sind, daß sie außer diesen Verbindungen Schichtgittersilikat und hochdisperses Siliciumdioxid in einer Gesamtmenge von 10-60 Gew.-%, bevorzugt 20-40 Gew.-%, enthalten, mit der Maßgabe, daß das Mengenverhältnis von Schichtgittersilikat zu Siliciumdioxid 20 zu 1 bis 0,25 zu 1 beträgt, und daß die Katalysatoren eine BET-Oberfläche von 5 bis 50 $m^2$/g, ein Makroporen-Volumen von 0,1 bis 0,8 $cm^3$/g und einen mittleren Porenradius von 1 bis $8 \cdot 10^{-7}$ cm aufweisen. Diese Katalysatoren zeigen eine ausgezeichnete Selektivität und ergeben gute Ausbeuten und Raum-Zeit-Ausbeuten. Sie sind hervorragend für die Anwendung in technischem Maßstab geeignet. Besonders vorteilhaft sind Katalysatoren, die Antimon, Vanadin und Titan enthalten.

Zur Herstellung der erfindungsgemäßen Katalysatoren werden Antimon und Vanadin sowie die Elemente Eisen, Kupfer, Titan, Kobalt, Mangan und Nickel zweckmäßigerweise als Verbindungen mit Sauerstoff, in elementarer Form oder als Verbindungen, die sich in Verbindungen mit Sauerstoff überführen lassen, vorzugsweise als Oxide, als Ammoniumsalze ihrer Sauerstoffsäuren oder als Nitrate, angewendet.

Die Mengenverhältnisse werden so gewählt, daß in den Katalysatoren der atomare Anteil an Antimon größer ist als der an Vanadin. Die atomaren Verhältnisse Antimon zu Vanadin liegen zweckmäßigerweise zwischen 1,1 zu 1 und 50 zu 1, vorzugsweise zwischen 1,1 zu 1 und 25 zu 1. Als atomare Verhältnisse Antimon zu Eisen, Kobalt, Kupfer, Mangan und Nickel einzeln oder gemeinsam kommen 2 zu 1 bis 20 zu 1, vorzugsweise 3 zu 1 bis 10 zu 1, in Frage. Jedoch sollen die atomaren Anteile an Eisen, Kobalt, Kupfer, Mangan und Nickel einzeln oder gemeinsam nicht den Anteil an Vanadin übersteigen. Als atomare Verhältnisse Antimon zu Titan sind 1 zu 3 bis 8 zu 1, vorzugsweise 1 zu 2 bis 4 zu 1, geeignet.

Den so zusammengesetzten Katalysatorsubstanzen wird erfindungsgemäß eine Mischung aus Schichtgittersilikat und hochdispersem Siliciumdioxid zugesetzt, so daß in den Katalysatoren deren Anteil 10 bis 60 Gewichtsprozent, vorzugsweise 20 bis 40 Gewichtsprozent, beträgt. Das Mengenverhältnis Schichtgittersilikat zu hochdispersem Siliciumdioxid beträgt in Gewichtsteilen 20 zu 1 bis 0,25 zu 1, vorzugsweise 10 zu 1 bis 1 zu 1.

In der Natur vorkommendes Schichtgittersilikat bedarf für die erfindungsgemäße Verwendung im allgemeinen einer Vorbehandlung. Es wird fein gepulvert und, zweckmäßigerweise unter ständiger Bewegung, beispielsweise in einem Drehrohr- oder Wirbelschichtofen, auf Temperaturen zwischen 900 und 1 200 °C erhitzt. Die Erhitzungszeit richtet sich nach der Art des Schichtgittersilikats, nach der Temperatur und nach der Art des Ofens. In den meisten Fällen wird die Substanz wenigstens eine Stunde, jedoch nicht mehr als 10 Stunden, auf Temperaturen in dem genannten Bereich gehalten. Bevorzugt wird als Schichtgittersilikat Montmorillonit und für diesen eine Behandlungszeit von 4 bis 6 Stunden bei 975 bis 1 050 °C.

Das hochdisperse Siliciumdioxid kann auf beliebige Weise gewonnen sein, beispielsweise durch Pyrolyse von Siliciumverbindungen oder durch Ausfällung aus Lösungen von Siliciumverbindungen. Es hat zweckmäßigerweise eine BET-Oberfläche etwa von 50 bis 500 $m^2/g$, vorzugsweise von 100 bis 300 $m^2/g$.

Zur Herstellung der erfindungsgemäßen Katalysatoren werden die Ausgangssubstanzen in möglichst fein verteilter Form innig vermischt. Es hat sich als vorteilhaft erwiesen, hierbei Wasser zuzusetzen und gegebenenfalls eine oder mehrere der Substanzen als Lösung oder Aufschlämmung in Wasser einzubringen. Den Mischungen werden Preßhilfsmittel sowie Porenbildner in möglichst fein verteilter Form und erforderlichenfalls weitere Flüssigkeiten, gegebenenfalls auch Trägersubstanzen, zugesetzt.

Als Preßhilfsmittel und Porenbildner werden für diese Zwecke übliche Substanzen angewendet, als Preßhilfsmittel beispielsweise Graphit, Stearinsäure oder Polyäthylenpulver, als Porenbildner beispielsweise Harnstoff, Ammoniumcarbonat oder Kohlehydrate, wie Saccharide, Stärke oder Cellulose. Der Gehalt der Katalysatormischung beträgt zweckmäßigerweise an Preßhilfsmitteln etwa 1 bis 15 Gewichtsprozent, vorzugsweise 2 bis 10 Gewichtsprozent, und an Porenbildnern etwa 0,1 bis 50 Gewichtsprozent, vorzugsweise 0,5 bis 40 Gewichtsprozent.

Als Flüssigkeiten kommen neben Wasser vornehmlich mit Wasser mischbare organische Lösungsmittel, insbesondere mehrwertige Alkohole, wie Glykol oder Glycerin, oder auch Mischungen dieser Flüssigkeiten in Frage. Der Gehalt der Katalysatormischung an Flüssigkeit beträgt zweckmäßigerweise etwa 10 bis 35 Gewichtsprozent, vorzugsweise 15 bis 30 Gewichtsprozent.

Bevorzugte Arbeitsweisen für die Bereitung der Katalysatormischungen sind, entweder zunächst Antimon oder Antimontrioxid in Salpetersäure einzusetzen und bei Siedetemperatur zu behandeln und danach Divanadinpentoxid oder Ammoniumvanadat und die anderen Elemente, diese als Nitrate beziehungsweise das Titan als Dioxid, sowie das Schichtgittersilikat und das hochdisperse Siliciumdioxid zuzufügen und erneut die Gesamtmischung bei Siedetemperatur zu behandeln oder diese Substanzen gleichzeitig in Salpetersäure einzusetzen und bei Siedetemperatur zu behandeln, schließlich, gegebenenfalls nach Neutralisation der Säure, die Mischungen zur Trockne zu bringen und auf Temperaturen von etwa 280 bis 300 °C zu erhitzen. Den so behandelten Mischungen wird das Preßhilfsmittel und der Porenbildner zugesetzt und, erforderlichenfalls nach Mahlung der Mischungen, zweckmäßigerweise auf Körnungen unter 0,5 mm, die Flüssigkeit zugefügt.

Die Katalysatormischungen werden dann zu Formlingen gepreßt, deren Größe zweckmäßigerweise etwa zwischen 1 und 8 mm liegt. Hierfür werden übliche Einrichtungen, beispielsweise Tablettiermaschinen oder Strangpressen, verwendet. Besonders geeignet sind Granulatformmaschinen, insbesondere Zahnrad-Granulatformmaschinen.

Die Formlinge werden in Gegenwart von Sauerstoff bei Temperaturen etwa zwischen 350 und 900 °C, vorzugsweise zwischen 500 und 800 °C, behandelt.

Die fertigen Katalysatoren haben im allgemeinen eine BET-Oberfläche etwa von 5 bis 50 $m^2/g$, ein Makroporen-Volumen etwa von 0,1 bis 0,8 $cm^3/g$ und einen mittleren Porenradius etwa von 1 bis $8 \cdot 10^{-7}$ cm. Ihre Schüttdichte ist etwa 0,9 bis 1,4 kg/l. Sie werden je nach Form und Größe im Festbett oder im Wirbelbett angewendet.

Die Umsetzung des 3-Methylpyridins mit Ammoniak und Sauerstoff zum 3-Cyanpyridin erfolgt in üblicher Weise in der Gasphase. Für die Wahl der Umsetzungsbedingungen ist ein breiter Spielraum gegeben. Die Umsetzung wird vornehmlich ohne Anwendung von Druck oder unter geringem Überdruck bis zu etwa 3 bar bei Temperaturen etwa zwischen 320 und 460 °C, vorzugsweise bei Temperaturen zwischen 340 und 440 °C, vorgenommen. Mit Vorteil wird der benötigte Sauerstoff als Luft zugeführt und den Gasen außerdem Wasserdampf zugemischt. Das Mengenverhältnis 3-Methylpyridin zu Ammoniak, Sauerstoff beziehungsweise Luft und gegebenenfalls Wasserdampf kann in weiten Grenzen gewählt werden. Im allgemeinen ist es zweckmäßig, je Mol 3-Methylpyridin etwa 2 bis 10 Mol, vorzugsweise 3 bis 8 Mol, Ammoniak, etwa 20 bis 40 Mol, vorzugsweise 25 bis 35 Mol, Luft und etwa 2 bis 10 Mol, vorzugsweise 3 bis 8 Mol, Wasserdampf anzuwenden. Je Liter Schüttvolumen des Katalysators und Stunde werden zweckmäßigerweise etwa 1 bis 2 Mol 3-Methylpyridin in den Reaktor eingespeist.

In den Beispielen bedeutet % Gewichtsprozente, soweit nicht anders bezeichnet.

Beispiel 1

23,3 kg Antimontrioxid, 4,7 kg Ammoniummetavanadat, 12,8 kg Titandioxid, 11,7 kg Montmorillonit und 5,8 kg hochdisperses Siliciumdioxid mit einer Oberfläche von 200 $m^2/g$ wurden in 140 l Wasser aufgeschlämmt. Dann wurden 16,4 l 54 %ige Salpetersäure zugesetzt. Die Mischung wurde langsam auf Siedetemperatur erwärmt, mit 7 l Wasser versetzt und 2 Stunden lang auf Siedetemperatur gehalten, danach mit Ammoniak auf den pH-Wert 4,6 eingestellt, abgekühlt, auf einem Walzentrockner getrocknet, in einem Drehrohrtrockner auf 300 °C erhitzt und in einer Stiftmühle auf eine Korngröße unter 0,5 mm vermahlen. 4 500 g der so bereiteten Katalysatormischung wurden mit 225 g Graphit und 1 700 g einer 20 %igen wäßrigen Harnstofflösung innig vermischt und dann zu Strangpreßlingen von 3 mm Durchmesser verformt. Die Preßlinge wurden im Luftstrom erhitzt und hierbei 15 Stunden auf 120 °C, 2 Stunden auf 550 °C, 1 Stunde auf 650 °C und 3 Stunden auf 770 °C gehalten. Die Schüttdichte des Katalysators war 1,05 kg/l, die BET-Oberfläche 18 m²/g, das Makroporen-Volumen 0,28 cm³/g und der mittlere Porenradius $2,7 \cdot 10^{-7}$ cm.

1 050 g des Katalysators wurden in ein Reaktionsrohr von 20 mm lichter Weite und 3 000 mm Länge gefüllt. Stündlich wurden in das Rohr in gleichförmigen Strom 1,34 Mol 3-Methylpyridin mit einem Gasgemisch eingespeist, das je Mol 3-Methylpyridin 6 Mol Ammoniak, 30 Mol Luft und 6 Mol Wasserdampf enthielt. Das Gasgemisch wurde dem Reaktionsrohr vorgewärmt zugeführt. Das Rohr wurde durch eine Salzschmelze beheizt, die auf 350 °C gehalten wurde. Bei Austritt aus dem Reaktionsrohr wurden die Gase mit Wasser gewaschen. Im Verlauf von 8 Stunden wurden im Durchschnitt 94 % des eingesetzten 3-Methylpyridins umgesetzt. Es betrug im Durchschnitt die Ausbeute an 3-Cyanpyridin, bezogen auf das eingesetzte 3-Methylpyridin, 89 Mol-% und die Raum-Zeit-Ausbeute 124 g/l.h.

Beispiel 2

Es wurde ein gleicher Katalysator verwendet und in gleicher Weise verfahren wie nach Beispiel 1, jedoch wurde die Salzschmelze auf 360 °C gehalten. Im Verlauf von 8 Stunden war im Durchschnitt der Umsatz 96 %, die Ausbeute an 3-Cyanpyridin 93 Mol-% und die Raum-Zeit-Ausbeute 129 g/l.h.

Beispiel 3

Es wurde ein gleicher Katalysator verwendet und in gleicher Weise verfahren wie nach Beispiel 1, jedoch wurde die Salzbadtemperatur auf 365 °C gehalten, und es wurden stündlich 1,47 Mol 3-Methylpyridin mit dem Gasgemisch eingespeist. Im Verlauf von 8 Stunden war im Durchschnitt der Umsatz 94 %, die Ausbeute an 3-Cyanpyridin 90 Mol-% und die Raum-Zeit-Ausbeute 137 g/l.h.

Beispiel 4

Es wurde ein gleicher Katalysator verwendet und in gleicher Weise verfahren wie nach Beispiel 1, jedoch wurde die Salzschmelze auf 365 °C gehalten und ein Gasgemisch eingespeist, das je Mol 3-Methylpyridin nur 4 Mol Ammoniak enthielt. Im Verlauf von 8 Stunden war im Durchschnitt der Umsatz 93 %, die Ausbeute an 3-Cyanpyridin 89 Mol-% und die Raum-Zeit-Ausbeute 124 g/l.h.

**Patentansprüche**

1. Katalysatoren für die Umsetzung von 3-Methylpyridin mit Ammoniak und Sauerstoff zu 3-Cyanpyridin, bestehend aus Verbindungen der Elemente Antimon, Vanadin und Sauerstoff und mindestens einem der Elemente Eisen, Kupfer, Titan, Kobalt, Mangan und Nickel, wobei das atomare Verhältnis Antimon zu Vanadin größer als 1 ist, dadurch gekennzeichnet, daß sie außer diesen Verbindungen Schichtgittersilikat und hochdisperses Siliciumdioxid in einer Gesamtmenge von 10-60 Gew.%, bevorzugt 20-40 Gew.%, enthalten, mit der Maßgabe, daß das Mengenverhältnis von Schichtgittersilikat zu Siliciumdioxid 20 zu 1 bis 0,25 zu 1 beträgt, und daß die Katalysatoren eine BET-Oberfläche von 5 bis 50 m²/g, ein Makroporen-Volumen von 0,1 bis 0,8 cm³/g und einen mittleren Porenradius von 1 bis $8 \cdot 10^{-7}$ cm aufweisen.

2. Verfahren zur Herstellung von Katalysatoren nach Anspruch 1, gekennzeichnet durch Bereitung von Mischungen, die Antimon und Vanadin im atomaren Verhältnis von größer als 1 und mindestens eines der Elemente Eisen, Kupfer, Titan, Kobalt, Mangan und Nickel als Verbindungen oder in elementarer Form und zusätzlich Schichtgittersilikat, hochdisperses Siliciumdioxid, Preßhilfsmittel und Porenbildner enthalten, und durch Erhitzen der Mischungen auf Temperaturen zwischen 350 und 900 °C in Gegenwart von Sauerstoff.

3. Verwendung der Katalysatoren nach Anspruch 1 oder 2 zur katalytischen Umsetzung von 3-Methylpyridin mit Ammoniak und Sauerstoff zu 3-Cyanpyridin.

4. Verfahren zur Herstellung von 3-Cyanpyridin durch katalytische Umsetzung von 3-Methylpyridin, dadurch gekennzeichnet, daß ein Katalysator gemäß Anspruch 1 oder 2 verwendet wird.

5. Verfahren nach Anspruch 4, dadurch gekennzeichnet, daß je Mol 3-Methylpyridin etwa 2 bis 10 Mol Ammoniak, etwa 20 bis 40 Mol Luft und etwa 2 bis 10 Mol Wasserdampf eingespeist werden und die Umsetzung bei Temperaturen etwa zwischen 320 und 460 °C erfolgt.

**Claims**

1. Catalysts for the reaction of 3-methylpyridine with ammonia and oxygen to produce 3-cyanopyridine, consisting of compounds of the elements antimony, vanadium and oxygen and at least one of the elements iron, copper, titanium, cobalt, manganese and nickel, the atomic ratio of antimony to vanadium being greater than 1, characterised in that, in addition to these compounds, they also contain lattice layer silicate and highly-dispersed silicon dioxide in a total quantity of from 10 to 60 % by weight, preferably from 20 to 40 % by weight, with the proviso that the ratio of lattice layer silicate to silicon dioxide is from 20 : 1 to 0 : 25 : 1, and that the catalysts have a BET surface area of from 5 to 50 m²/g, a macropore volume of from 0.1 to 0.8 cm³/g and an average pore radius of from 1 to $8 \cdot 10^{-7}$ cm.

2. A process for the production of catalysts according to claim 1, characterised by the preparation of mixtures containing antimony and vanadium in the atomic ratio of greater than 1 at least one of the elements iron, copper, titanium, cobalt, manganese and nickel as compounds or in elementary form and in addition lattice layer silicate, highly-dispersed silicon dioxide, pressing auxiliaries and pore-formers, and by heating the mixtures to temperatures of from 350 °C to 900 °C in the presence of oxygen.

3. The use of catalysts according to claim 1 or claim 2, for the catalytic reaction of 3-methylpyridine with ammonia and oxygen to produce 3-cyanopyridine.

4. A process for the production of 3-cyanopyridine by the catalytic reaction of 3-methylpyridine, characterised in that a catalyst according to claim 1 or claim 2 is used.

5. A process according to claim 4, characterised in that about 2 to 10 mols of ammonia, about 20 to 40 mols of aire and about 2 to 10 mols of steam are fed in per mol of steam and the reaction is carried out at a temperature of from about 320 °C to 460 °C.

**Revendications**

1. Catalyseurs pour la transformation de 3-méthylpyridine avec l'ammoniac et de l'oxygène en 3-cyanopyridine, constitués de composés des éléments antimoine, vanadium et oxygène, et au moins un des éléments fer, cuivre, titane, cobalt, manganèse et nickel, le rapport atomique de l'antimoine au vanadium étant supérieur à 1, catalyseurs caractérisés en ce que, outre ces composés, ils contiennent des silicates stratifiés poreux et du dioxyde de silicium à dispersion élevée, dans une proportion globale de 10-60 % en poids, de préférence 20-40 % en poids, avec cette condition que le rapport des silicates stratifiés poreux au dioxyde de silicium est compris entre 20 pour 1 et 0,25 pour 1, et que les catalyseurs présentent une surface BET de 5 à 50 m$^2$/g, un volume des macropores de 0,1 à 0,8 cm$^3$/g et un rayon moyen des pores de 1 à 8 · 10$^{-7}$ cm.

2. Procédé de préparation de catalyseurs selon la revendication 1, caractérisé par la préparation de mélanges contenant de l'antimoine et du vanadium dans un rapport atomique supérieur à 1 et au moins un des éléments fer, cuivre, titane, cobalt, manganèse et nickel, sous forme de composés ou sous forme élémentaire, et en plus, des silicates stratifiés poreux, du dioxyde de silicium à dispersion élevée, des auxiliaires de pressage et des formateurs de pores, et chauffage du mélange à une température comprise entre 350 et 900 °C, en présence d'oxygène.

3. Utilisation des catalyseurs selon les revendications 1 ou 2 pour la transformation catalytique de 3-méthylpyridine, avec de l'ammoniac et de l'oxygène, en 3-cyanopyridine.

4. Procédé de préparation de 3-cyanopyridine par réaction catalytique de 3-méthylpyridine, caractérisé en ce qu'un catalyseur selon les revendications 1 ou 2 est utilisé.

5. Procédé selon la revendication 4, caractérisé en ce que par mole de 3-méthylpyridine, on introduit environ 2 à 10 moles d'ammoniac, 20 à 40 moles, d'air, et 2 à 10 moles de vapeur d'eau et que la réaction s'effectue à des températures comprises environ entre 320 et 460 °C.